# EUROPEAN PATENT APPLICATION

(11) **EP 1 752 042 A1**
(43) Date of publication of application: **14.02.2007**
(21) Application number: 05734126.5
(22) Date of filing: 21.04.2005
(51) Int. Cl.: A01N 1/00, A61G 17/00

(54) **APPARATUS FOR CLOSING BODY CAVITY OF DEAD BODY AND METHOD OF TREATING DEAD BODY**

(30) Priority: 21.05.2004 JP 2004152297
(71) Applicant: Nishihara, Risa, Hiroshima 730-0005 (JP)
(72) Inventor: OKAMOTO, Toshiki, Hiroshima-shi, Hiroshima 733-0012 (JP)
(74) Representative: Thoma, Michael
(86) International application number: PCT/JP2005/007640
(87) International publication number: WO 2005/112625

(57) **Abstract**

This invention aims at developing a sanitary body cavity blocking device which prevents leakage of substances in the rectum of a corpse due to anal muscle relax after die, simplifies the postmortem treatment work for persons working on it or nurses and prevents infection to the persons, nurses or those around due to leakage of body internal substances. An expandable sealing member (2) is made of porous fiber elements that absorb water on contact with body fluids in a body cavity to expand up to approximately 2.5 or more times its diameter. The expandable sealing member (2) is inserted, in the form loaded in a cylindrical member (3), into a body cavity such as an anus, pushed out of the cylindrical member (3) by a rod member (10) to reside in the rectum, rapidly absorbs water to swell, comes into tight contact with the inner wall of the rectum, further expands the rectum and seals the passage of the rectum.

## Description

### Technical Field

This invention relates to body cavity blocking devices for preventing leakage of internal substances from body cavities of a corpse and its attendant contamination to the surroundings and infection to those around and corpse treatment devices using the same, and particularly relates to a body cavity blocking device for preventing leakage of internal substances from cavities of a lower body, such as the anus or vagina, and its attendant contamination to the surroundings and infection to those around and a corpse treatment device using the same.

### Background Art

Usually, after humans die, muscles of their body cavities become flaccid so that waste materials in the body cavities often leak out. Further, because of intravenous drip during medical treatment in life, the bodies after death often contain a large amount of body fluids. In particular, as for a body cavity in their low bodies, i.e., the anus, the anal muscle is relaxed to open the anus, resulting in leakage of internal waste materials. Furthermore, if they are female, the vaginal cavities often leak out a large amount of body fluids. To prevent these leakages, the anus and vaginal cavity is filled with a huge amount of absorbent cotton before the occurrence of postmortem rigidity.

Such work is often carried out by a nurse. Not only the work is troublesome and unsanitary but also the worker at work may be infected with germs or viruses from the leaked substances. Therefore, there is a strong need to surely prevent such infection. Traditional countermeasures to the infection, however, are not good in terms of feasibility.

For example, a diaper is used in many cases in order to prevent leakage of internal waste materials in cavities of parts of a corpse, particularly, in cavities of the lower body such as the anus or vaginal cavity. In these cases, the diaper cannot fully prevent the leakage of waste materials, leading to contamination due to leakage. In addition, the nurse inevitably contacts the waste materials during diaper cleaning for replacement. For these reasons, if the body is a germ carrier, those around may be infected.

Techniques are known which eliminate the above problems and prevent leakage of internal substances in corpse lower body cavities, such as the anus or vaginal cavity, and in which body cavity sealing devices are directly inserted into lower body cavities, such as the rectum or vaginal cavity. (See, for example, Patent Documents 1 and 2)
As shown in Figure **17,** the body cavity sealing device in Patent Document 1 is designed so that a plug part **(101)** is inserted into the anus, a gas injector **(Y)** is then inserted into an opening **(108)** located at a joint **(A)** between the plug part **(101)** and an anus abutment member **(102)** and the plug part **(101)** is charged with gas. The gas flows from the inside of the plug part **(101)** through a gas guide hole **(106)** to open a non-return valve **(107),** is fed to an control chamber **(105)** and expands the control chamber **(105).** Thus, a flexible member **(104)** comes into tight contact with the inner wall of the anus to seal the anus. Reference numeral **112** denotes a lubricant layer.

As shown in Figures **18** and **19,** the body cavity sealing device **200** in Patent Document 2 is composed of a columnar superabsorbent fiber molded object **201,** a water-soluble sheet **202** sheathing the molded object **201,** and a lubricant **203** covering the sheet **202.** The body cavity sealing device **200** has one end tapered to facilitate its insertion. The superabsorbent fiber molded object **201** disclosed in the publication is made of water-swellable fibers (trade name: LANSEAL®F manufactured by Toyobo Co., Ltd.) of double-layer structure that has an inner layer made of an acrylic fiber serving as a core and a superabsorbent outer layer placed to surround the inner layer and processed to swell after absorbing water. The body cavity sealing device **200** is enveloped in a bag **204** made of a protective covering material.
Patent Document 1: Japanese Unexamined Patent Publication No. 2001-161733
Patent Document 2: Japanese Unexamined Patent Publication No. 2003-111830

### Disclosure of the Invention

### Problems to Be Solved by the Invention

As for the body cavity sealing device **100** in Patent Document 1, the plug part **(101)** is inserted in the anus and part of the anus then fills in between the expanded control chamber **(105)** and the anus abutment member **(102).** This prevents the plug part **(101)** from entering deep in the anus or getting out of the anus.

Since, however, the anus abutment member **(102)** protrudes from the anus and is exposed to the outside, the body does not have a natural appearance and shows up that it has undergone some kind of treatment. Therefore, the use of this device is often rejected by bereaved families or nurses who treat bodies. Further, the above device involves the use of a tool Y for expanding it with gas and also needs to be sealed against leakage of the gas. Therefore, the device is relatively hard to handle as a body cavity sealing device and cannot be said to have a sufficient anti-leakage function. In particular, since the device is not of a type that expands by absorbing body fluids, it may leak out body fluids if even a slight clearance exists therein, which does not provide a sufficient function as a body cavity sealing device. In addition, the control of gas pressure is difficult.

In using the body cavity sealing device **200** in Patent Document 2, the bag **204** is first opened to take out the body cavity sealing device **200.** Then, the tapered side of the sealing device **200** is oriented to the anus of a body and, in this position, the sealing device **200** is pushed through the anus into the rectum. Since the body cavity sealing device **200** is pushed through the anus in the rectum, it cannot be seen from the outside of the body. Therefore, the use of this device will not be rejected by bereaved families or others concerned on the ground of its appearance. Further, the body cavity sealing device **200** is formed so that a superabsorbent fiber molded object **201** is wrapped in a water-soluble sheet **202** and a lubricant **203** such as glycerin is further applied to the outer surface of the sheet **202.** In this manner, this device is contrived to easily slide in the anus when forcedly inserted through the anus.

This device, however, may not enter through the anus into the rectum well depending upon individual skill levels of nurses or others handling it. The reason is believed to be as follows: Even if the fiber molded object **201** is wrapped in the sheet **202** and the lubricant **203** is applied to the sheet **202,** both the layers of the sheet **202** and the lubricant **203** are thin and soft. Therefore, on contact with muscles of the anus inner wall, such a thin and soft surface cannot slide on the muscles well to enter the rectum. Further, in order to insert the molded object deeply to the rectum, the worker needs to put his or her finger in the anus and, in this state, push the molded object inwardly. Therefore, this is a reluctant work for the workers even if they wear gloves.

The present invention has been made in view of the foregoing points and, therefore, its object is to provide a sanitary body cavity blocking device which prevents leakage of substances in cavities of the lower part of a corpse, such as the rectum and vaginal cavity, simplifies the body treatment work for persons working on it and nurses and prevents infection to the persons, nurses or those around due to leakage of body internal substances. Further, another object of the present invention is to provide a corpse treatment device in the form of a kit including not only a body cavity blocking device for sealing a cavity in the lower part of a corpse but also anti-leakage members for other body cavities, in particular, anti-leakage members for preventing leakage of body fluids from the throat and mouth.

### Means to Solve the Problems

The body cavity blocking device of the present invention is a corpse body cavity blocking device comprising an expandable sealing member and an insertion member for insertion of the expandable sealing member into a body cavity of a corpse, wherein the expandable sealing member is a molded object obtained by bundling fiber elements of double-layer structure into a substantially columnar shape and is configured, when inserted into the body cavity of the corpse, to absorb body fluids to expand thereby sealing the passage of the body cavity and preventing the leakage of the body fluids, each of the fiber elements includes an inner layer which is a core made of an acrylic fiber and an outer layer which is formed to surround the inner layer and has a higher water-absorbability and higher swellability than the inner layer, and has the property of absorbing body fluids in the body cavity to expand up to 2.5 or more times the diameter thereof, the insertion member comprises: a cylindrical member having an insertion head with which the cylindrical member is first inserted into the body cavity and containing the expandable sealing member slidably placed in the cylindrical member; a disc-shaped push member placed in the cylindrical member and at one end of the expandable sealing member to slide inside the cylindrical member in the axial direction of the cylindrical member; and a rod member provided at the opposite side of the disc-shaped push member to the expandable sealing member to cause axial sliding movement of the disc-shaped push member inside the cylindrical member, the insertion head of the cylindrical member is provided with a plurality of flexible pinnate members formed to extend toward the central axis of the cylindrical member while curving, the rear end of the cylindrical member is provided integrally with a resistant part which extends from the inner surface of the cylindrical member toward the central axis thereof and provides a resistance to the sliding out of the disc-shaped push member from the rear end of the cylindrical member, and the rod member is longer than the cylindrical member.

Preferably, the outer layer of each of the fiber elements is made of hydrophilic cross-linked polymer obtained by hydrophilically cross-linking an acrylic fiber and each of the fiber elements has the property of absorbing body fluids to expand up to five or more times the diameter thereof and has a degree of water swelling of 5 to 200 cc/g.

The resistant part is preferably a raised part formed to extend 0.05 to 0.5 mm from the inner surface of the cylindrical member toward the central axis thereof.

A flange for the pushing of the rod member is preferably provided at the end of the rod member opposite to the disc-shaped push member.

The flange preferably has the same shape as the disc-shaped push member.

Preferably, the disc-shaped push member comprises a disc part contacting the expandable sealing member and a ring part slidably contacting the inner surface of the cylindrical member, the ring part extends from the peripheral edge of the disc part toward the rear end of the cylindrical member, and the connecting part between the disc part and the ring part is chamfered.

Preferably, the disc-shaped push member has a fitting projection formed integrally on the surface thereof opposite to the surface thereof contacting the expandable sealing member, and the rod member has a cylinder whose outer diameter is smaller than the inner diameter of the cylindrical member so that the fitting projection is fitted into the inner surface of an end part of the cylinder.

In a preferred embodiment, the outer periphery of the cylindrical member is formed with a mark indicating a target distance by which the cylindrical member should be inserted into the body cavity.

In a preferred embodiment, the cylindrical member is formed to have a larger wall thickness in a portion close to the rear end thereof than in a portion close to the insertion head thereof, and the portion of the cylindrical member close to the insertion head is formed to have the same inner diameter as, but a smaller outer diameter than the portion of the cylindrical member close to the rear end.

In a preferred embodiment, the outer periphery of the cylindrical member is provided with a stopper for restricting the distance of insertion of the cylindrical member.

Preferably, an opening is formed at the insertion head of the cylindrical member, and the diameter of the opening is 0.7 to 0.3 times as large as the inner diameter of the cylindrical member.

The corpse treatment device of the present invention comprises: the above body cavity blocking device; a syringe filled with a jelly substance which contains superabsorbent resin and is to be infused into the throat of a corpse; an insertion tube connectable to an infusion head of the syringe; a cover member for covering the insertion tube; a cotton piece for a mouth to which a jelly substance containing superabsorbent resin is adhered; and a container for containing the body cavity blocking device, the syringe, the insertion tube, the cover member and the cotton piece, wherein the insertion tube comprises: a tube body which is made of flexible synthetic resin and configured to be inserted through a nostril into a throat; and a connecting part provided at the rear end of the tube body and connectable to the syringe, and has a tube opening formed at the front end of the tube body.

Gloves for handling a corpse are preferably further contained in the container.

A paper sheet for wiping hands is preferably further contained in the container.

Sealing members for an earplug and a nose plug are preferably further contained in the container.

A vessel containing a lubricant is preferably further contained in the container.

A container bag for containing the used components including the body cavity blocking device, the syringe, the insertion tube and the cover member is preferably further contained in the container.

### Effects of the Invention

Since the body cavity blocking device includes the insertion member, the expandable plug member can be easily inserted into a body cavity of a corpse to fully get in it. Once inserted, the expandable plug member immediately absorbs body fluids and expands to come into tight contact with the inner wall of the body cavity. Therefore, the expandable plug member can be held in the body cavity and can surely prevent body fluids from leaking out of the body cavity.

In the corpse treatment device of the present invention, all necessary items are kept on hand in order to prevent leakage of body fluids from cavities of a corpse in treating the corpse, particularly, all items for treating cavities in a lower body, such as the anus or virginal cavity, the throat and the mouth are kept on hand. Therefore, the treatment for preventing leakage of body fluids from a corpse can be successfully implemented in a short time.

### Brief Description of the Drawings

[Fig. 1] Figure **1** shows a body cavity blocking device according to a first embodiment of the present invention.
[Fig. 2] Figure **2** is a front view of a cylindrical member in Figure **1** and shows flexible pinnate members.
[Fig. 3] Figure **3** is a cross-sectional view taken along the line X-X in Figure **1.**
[Fig. 4] Figure **4** is a partly enlarged cross-sectional view of the rear end of the cylindrical member in Figure **1.**
[Fig. 5] Figure **5** is a partly enlarged cross-sectional view of a porous fiber element of an expandable sealing member.
[Fig. 6] Figure **6** illustrates a condition of the body cavity blocking device according to the first embodiment when it is used in the rectum of a corpse.
[Fig. 7] Figure 7 is a view illustrating a condition of the body cavity blocking device after it is inserted in the rectum.
[Fig. 8] Figure **8** is a cylindrical member according to a second embodiment of the present invention.
[Fig. 9] Figure **9** is a cylindrical member according to a third embodiment of the present invention.
[Fig. 10] Figure **10** is a cylindrical member according to a fourth embodiment of the present invention.
[Fig. 11] Figure **11** shows an example of a corpse treatment device used with the corpse body cavity blocking device of the present invention.
[Fig. 12] Figure **12** shows equipment additionally used as a component of the corpse treatment device of the present invention.
[Fig. 13] Figure **13** shows another equipment additionally used as a component of the corpse treatment device of the present invention.
[Fig. 14] Figure **14** shows still another equipment additionally used as a component of the corpse treatment device of the present invention.
[Fig. 15] Figure **15** shows earplugs and nose plugs additionally used as components of the corpse treatment device of the present invention.
[Fig. 16] Figure **16** shows a lubricant.

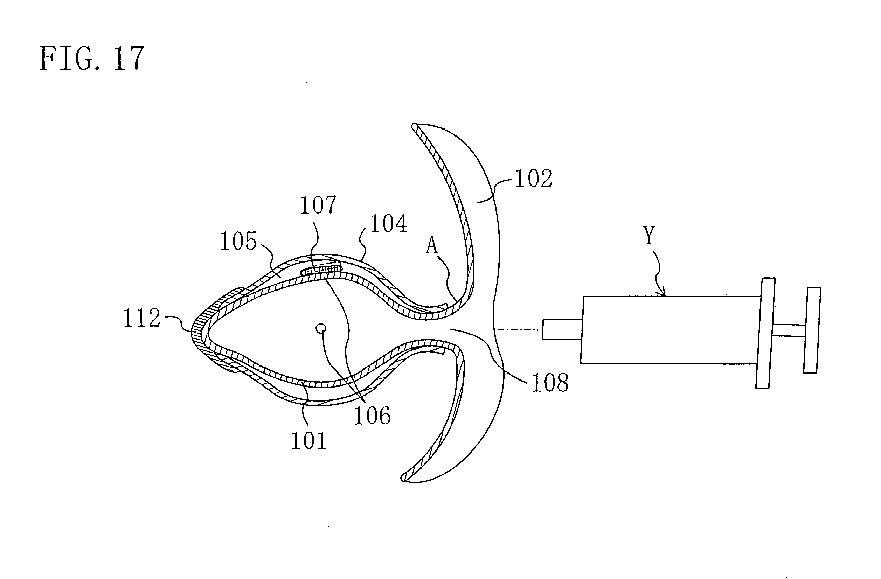

[Fig. 17] Figure **17** shows a known art.

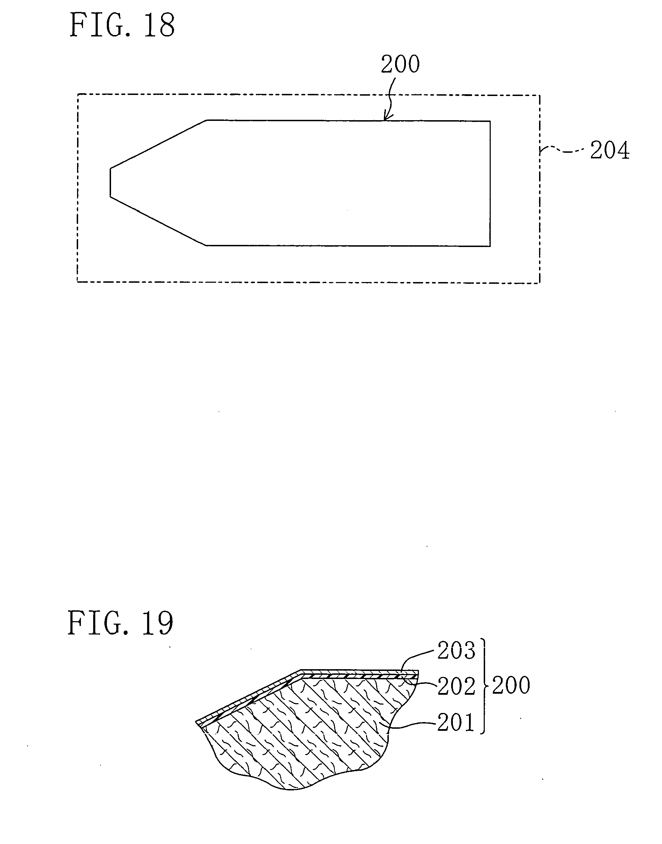

[Fig. 18] Figure **18** shows another known art.
[Fig. 19] Figure **19** shows a partly cross-sectional view of the known art shown in Figure **18.**

### Explanation of Reference Numerals

- 1: body cavity blocking device
- 2: expandable sealing member
- 2a: inner layer
- 2b: superabsorbent outer layer
- 3: cylindrical member
- 3a: rear end
- 4: flexible pinnate member
- 6: raised part
- 7: disc-shaped push member
- 8: ring part
- 10: rod member
- 11: flange
- 17: disc part
- 20: corpse treatment device
- 21: syringe
- 22: jelly substance
- 23: insertion tube
- 27: cover member
- 28: cotton piece
- 29: container
- 31: gloves
- 32: paper sheet
- 33: sealing member
- 34: vessel
- 35: lubricant
- 36: container bag
- 43: cylindrical member
- 53: cylindrical member
- 63: cylindrical member

### Best Mode for Carrying Out the Invention

Embodiments of the present invention will be described below in detail with reference to the drawings.

### (First Embodiment)

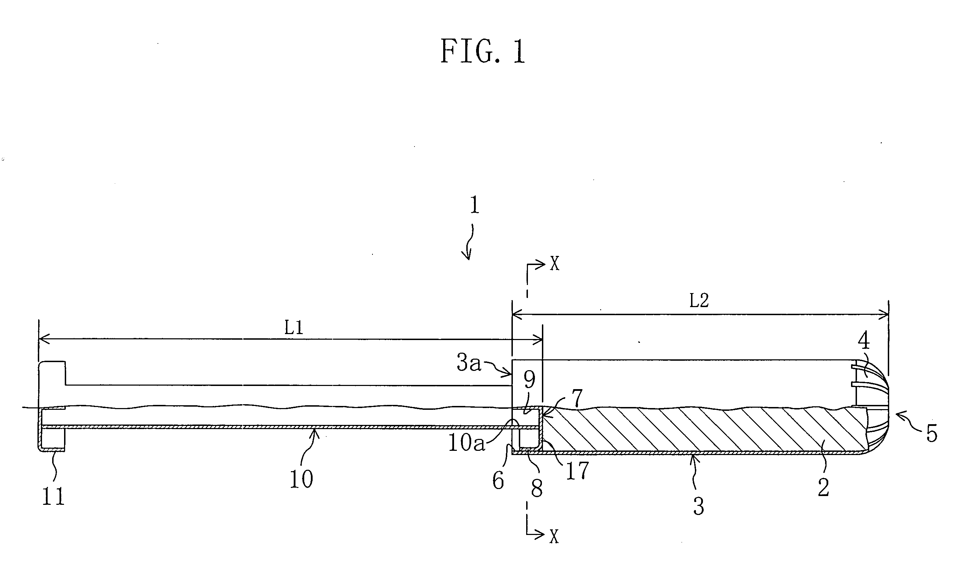

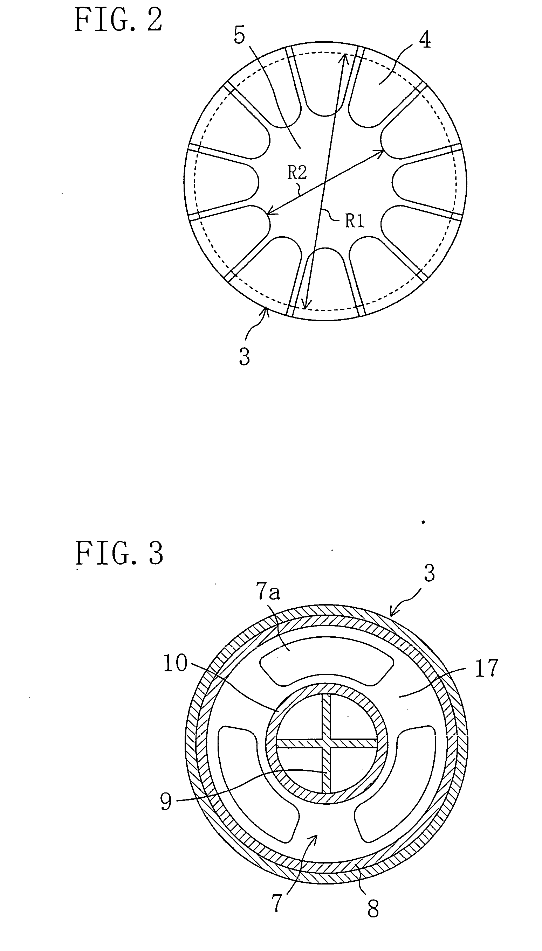

Figures 1 to 5 are drawings according to the first embodiment. Figure 1 shows a body cavity blocking device of the present invention, in which part thereof is shown in a cross-sectional view and the other is shown in a outside view. The body cavity blocking device **1** includes an insertion member and an expandable sealing member **2** loaded in the insertion member. The insertion member has a plastic cylindrical member **3.** A columnar expandable sealing member **2** is slidably contained in the cylindrical member **3.** A plurality of flexible pinnate members **4** are provided integrally at an insertion head of the cylindrical member 3 beginning with which the cylindrical member **3** is inserted into a body cavity.

The flexible pinnate members **4** are formed by a plurality of divided pieces to extend toward the cylinder central axis of the cylindrical member **3** while curving, and form a smooth, tapered shape to allow ease of insertion of the cylindrical member **3** into the anus. The tapered shape is a substantially semispherical and its insertion head formed by a curved surface allows ease of insertion into a body cavity. In this embodiment, the number of the flexible pinnate members **4** is twelve. A larger number of flexible pinnate members **4** is more preferable because this makes it easier for the expandable sealing member **2** to push the flexible pinnate members **4** open and to be then inserted into a body cavity. The number of flexible pinnate members **4** is preferably six or more and more preferably twelve or more.

An opening **5** is formed at the insertion head of the cylindrical member **3.** The diameter **R2** of the opening **5** is set at 0.7 to 0.3 times the inner diameter **R1** of the cylindrical member **3.** If the opening **5** is too large, a tapered guide part (insertion head) of the cylindrical member **3** falls short of mass when inserted into the body cavity. This makes it difficult to insert the cylindrical member **3** thereinto. On the other hand, if the opening is too small, it is necessary to open the flexible pinnate members **4** largely outwardly in pushing out the columnar molded part (expandable sealing member) contained in the cylindrical member **3.** In this case, the resistance to the pushing out becomes too great. Therefore, the diameter **R2** of the opening **5** is preferably set within the above range. In particular, it is more preferable to set the diameter **R2** of the **opening 5** at 0.62 to 0.46 times the inner diameter **R1** of the cylindrical member **3.**

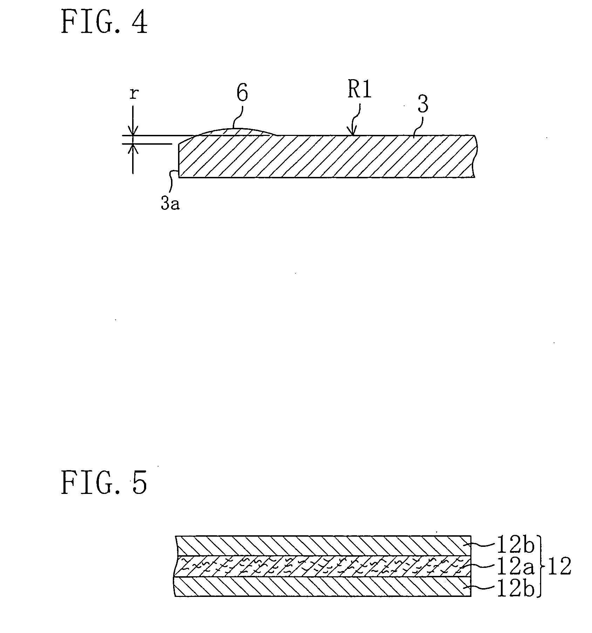

A plastic disc-shaped push member 7 is placed at the rear end of the expandable sealing member **2** (the end thereof opposite to the end thereof located toward the insertion head of the cylindrical member 3) for axial sliding movement inside the cylindrical member **3.** The disc-shaped push member 7 is formed of a disc part **17** brought to bear on the expandable sealing member **2** and a ring part **8** extending from the peripheral edge of the disc part **17** toward the rear end of the cylindrical member **3.** The disc part **17** and the ring part **8** are integrally formed with each other. The disc part **17** and the ring part **8** are substantially perpendicular to each other. The outside surface of the ring part **8** substantially entirely comes into contact with and slides on the inner surface of the cylindrical member **3.** The connecting part between the disc part **17** and the ring part **8** is chamfered. In this embodiment, the connecting part is chamfered to form a curved surface. In other words, as viewed in a cross section including the central axis of the cylindrical member **3,** a smooth curve continues from the disc part **17** to the ring part **18.** Thus, the disc-shaped push member 7 is formed to easily slide into the cylindrical member **3** when inserted thereinto.
The rear end of the cylindrical member **3** is formed integrally with a raised part **6** extending toward the central axis of the cylindrical member **3** to restrain the disc-shaped push member **3** form sliding out of the rear end **3a.** The raised part **6** is a circumferentially continuous rib provided on the inner surface of the cylindrical member **3** and, as shown in Figure **4,** is formed by a curve as viewed in a cross section including the central axis of the cylindrical member **3.** Its rear end is formed to slightly sink from the level of the inner wall surface of the cylindrical member **3** toward the outer wall surface thereof and its forward end terminates to have the same diameter as the inner diameter **R1** of the cylindrical member **3** at its inner wall surface level. In other words, in order that the disc-shaped push member **7** easily slides into the cylindrical member **3** when inserted thereinto, the rear end of the cylindrical member **3** is formed to have a slightly larger diameter than the inner diameter **R1** (a diameter difference: **r).** The raised part **6** is formed to protrude 0.05 to 0.5 mm from the inner surface of the cylindrical member **3** (a portion thereof having the inner diameter **R1)).** If the amount of protrusion of the raised part **6** is too small, this provides an insufficient function of preventing the push member **7** from sliding out of the cylindrical member **3.** If the amount of protrusion is too large, this makes it difficult to insert the push member **7** into cylindrical member **3.** Therefore, it is preferable to set the height of the raised part **6** within the above range. More preferably, the height of the raised part 6 is set at 0.1 to 0. 25 mm.

The raised part **6** is not limited to that as in this embodiment and its shape and size are not limited to the above so long as it can prevent the push member 7 from improperly sliding out after inserted in the cylindrical member **3,** i.e., so long as it functions as a resistant part providing a resistance to the sliding out of the push member **7.** For example, the rear end of the cylindrical member **3** may be formed with a thread-like raised part to insert the ring part into the cylindrical member **3** by screwing. Alternatively, the rear end of the cylindrical member **3** may be formed with a plurality of raised parts and corresponding recesses may be formed in the ring part. In this case, the push member 7 starts to be inserted into the cylindrical member **3** at the point where the raised parts are circumferentially aligned with the recesses, and is slightly turned away from the alignment after inserted therein, thereby preventing an improper sliding out.

A plastic rod member **10** for sliding the disc-shaped push member **7** in the axial direction of the cylindrical member **3** is provided on the center of the surface (outside surface) of the disc part **17** opposite to the surface thereof contacting the expandable sealing member **2.** The center of the outside surface of the disc part **17** is formed with a cross-shaped fitting projection **9** to form an integral piece with the disc-shaped push member **7.** The rod member **10** is formed of a hollow, elongated cylinder. The fitting projection **9** is configured to fit into the inner surface **10a** of the cylinder. The outer diameter of the cylinder is smaller than the inner diameter of the cylindrical member **3.** The rod member **10** is fitted on the fitting projection 9 and both the members are further joined together, for example, by fusion. Since the disk-like push member 7 and the rod member **10** are separately manufactured, these members can be made of individual suitable materials, which reduces material cost and production cost. Further, the disc-shaped push member **7** is formed with a plurality of holes **7a** serving as air releases when the expandable sealing member **2** is pushed into the cylindrical member **3.**

The shape of the fitting projection **9** is not limited to the cross shape but may be trifurcate or other shapes. The shape and size of each hole **7a** and the number of holes **7a** are not limited to those in this embodiment.

The rear end of the rod member **10** is formed integrally with a flange **11.** The flange **11** is formed in the same shape as that of the disc-shaped push member **7.** The ring part **8** of the disc-shaped push member **7** and the ring part of the flange **11** are fixed to the cylinder to face each other. Therefore, the flange **11** and the disc-shaped push member 7 are configured so that either member can be inserted into the cylindrical member 3. If the disc-shaped push member **7** and the flange **11** have different shapes, assembly error may occur when the body cavity blocking device is assembled. Since, in this embodiment, the disc-shaped push member 7 and the flange **11** have the same shape, assembly error does not occur thereby enhancing assembly workability. The flange **11** functions as a part to which a force is applied when the expandable sealing member **2** is inserted into a body cavity. The flange **11** also functions as a part with which a finger is engaged or which is held by fingers when the rod member **10** is pulled out of the body cavity together with the cylindrical member **3** after the expandable sealing member **2** is inserted therein.

The tail end of the ring part **8** of each of the disc-shaped push member **7** and the flange **11** (the other side of the ring part **8** from the connecting part with the disc part **17)** is an end surface perpendicular to the outside surface thereof and the corner formed by the end surface and the outside surface has a substantially right angle. Therefore, it is difficult that the flange **11** slides over the raised part **6** and enters the cylindrical member **3.** Specifically, since the outside corner at the tail end of the ring part **8** is substantially at a right angle, the corner engages the raised part 6 so that the flange **11** is hinged from entering the cylindrical member **3.** Therefore, even if the rod member **10** is excessively pushed in in inserting the expandable sealing member **2,** the flange **11** can be prevented from entering the cylindrical member **3.**

The length **L1** of the rod member **10** is set to be longer than the length **L2** of the cylindrical member **3** so that the rod member **10** can partly extend out of the cylindrical member **3** and be left outside even when pushed therein. The left part of the rod member **10** or the flange **11** can be held by hand in order to pull it out of the cylindrical member **3.**

The expandable sealing member (superabsorbent fiber molded object) **2** is a molded object obtained by bundling water-swellable fibers (fiber elements) **12** into a columnar shape, wherein each water-swellable fiber is of double-layer structure having an inner layer **12a** which is made of an acrylic fiber and serves as a core and an outer layer **12b** which has a higher water-absorbability than the inner layer **12a,** is placed to surround the inner layer and processed to swell after absorbing water. The expandable sealing member 2 employs as the superabsorbent fiber **12** a fiber that expands up to five or more times its diameter by absorbing water (trade name: LANSEAL®F manufactured by Toyobo Co., Ltd.). Both the expandable sealing members **2** used for a rectum and for a virginal cavity are the same and have a size of 20mm diameter and 80mm length. As the expandable sealing member **2** has a smaller diameter, it is more easily inserted through the anus **H** into the body cavity (rectum **G).** In this case, however, a problem arises that it takes long time until the expandable sealing member swells to expand up to a size that comes into tight contact with the rectum wall since it is inserted in the rectum. On the contrary, if the diameter of the body cavity sealing member is too large, it is hard to insert into the anus **H.** Therefore, the diameter of the expandable sealing member is preferably 10 to 25 mm. Further, as the expandable sealing member has a smaller length, it is more easily inserted into the rectum **G.** However, if the length of the expandable sealing member is too small, it comes short of the volume for swelling, thereby degrading its sealing function. Therefore, the length of the expandable sealing member is preferably 40 to 110 mm. More preferably, the length of the expandable sealing member is 60 to 90 mm.

Since the core of the inner layer **12a** of each of superabsorbent fibers **12,** which are components of the expandable sealing member **2,** keeps the shape of a fiber after absorbing water, the expandable sealing member **2** retains a substantially cylindrical shape also after expanded and is securely held in the body cavity. Further, when absorbing water to rapidly expand in the radial direction, the expandable sealing member **2** promptly comes into tight contact with the passage inner wall of the body cavity and exhibits sealing performance, which is advantageous. For this purpose, the superabsorbent fiber **12** used is preferably of a type that expands up to 2.5 or more times its diameter by absorbing water, more preferably of a type that expands up to five or more times its diameter, and still more preferably of a type that expands up to ten or more times its diameter. Since larger diameter expansion means higher water absorption capacity, more largely expandable fibers are preferable. In particular, the superabsorbent fiber is preferably of a type that has an inner layer **12a** made of acrylonitrile-based polymer and having a degree of water swelling of 5 to 200 cc/g. If the outer layer **12b** contains carboxyl groups, this is preferable because they have adsorbability to ammonia and therefore can absorb odor. The content of carboxyl groups is preferably 0.5 to 4.0 mmol/g. The outer layer **12b** of the superabsorbent fiber 12 is obtained by treating an acrylic fiber with aqueous alkali metal hydroxide solution to hydrophilically crosslink only the outer layer of the acrylic fiber. Through such a treatment, carboxyl groups in alkali metal salt form are introduced into the acrylic fiber.

A description will be made of the work for loading the expandable sealing member 2 **into** the cylindrical member **3** in the first embodiment. A columnar expandable sealing member **2** is loaded into the cylindrical member **3** through its opening at the rear end **3a.** In this case, though the raised part **6** is formed at the rear end **3a,** its height is too small to hinder the loading of the expandable sealing member **2.** Even if the diameter of the opening at the rear end **3a** is lightly smaller than the outer diameter of the expandable sealing member **2,** this does not hinder the loading of the expandable sealing member **2** because the expandable sealing member **2** is made of porous fiber elements having elasticity and therefore can be loaded into the cylindrical member **3** with a small force.

Thereafter, the rod member **10,** which has been integrally joined at both ends to the disc-shaped push members **7,** is inserted into the cylindrical member **3.** Though the inner diameter of the opening surrounded by the raised part **6** is slightly smaller than the outer diameter of the disc-shaped push member **7,** i.e., the outer diameter of the ring part **8,** the difference between both the diameters is small. Therefore, the rod member **10** can be pushed into the cylindrical member **3** by pushing the disc-shaped push member **7** and the ring part **8** with a small force, thereby providing a good workability. In particular, since the connecting part between the disc-shaped push member **7** and the ring part **8** is formed by a curved surface, it can go over the raised part **6** and enter inward with a small force. After inserted, the rod member **10** is stopped being inserted at a point where the disc-shaped push member **7** contacts the rear end surface of the expandable sealing member 2, thereby completing the insertion.

Since the expandable sealing member **2** is thus loaded into the cylindrical member **3** through the opening at the rear end **3a** which is a wide opening of the cylindrical member **3,** it can be loaded therein without the deformation of the outer periphery and without any resistance. In addition, since the disc-shaped push member **7** and the ring part **8** can be inserted into the cylindrical member **3** with a small force, the loading and insertion work is extremely easy to do and, therefore, provides excellent productivity. Further, since the raised part **6** restrains the disc-shaped push member 7 and the ring part **8** from improperly sliding out of the cylindrical member **3** during transportation and storage, the disc-shaped push member 7 is prevented from dropping out.

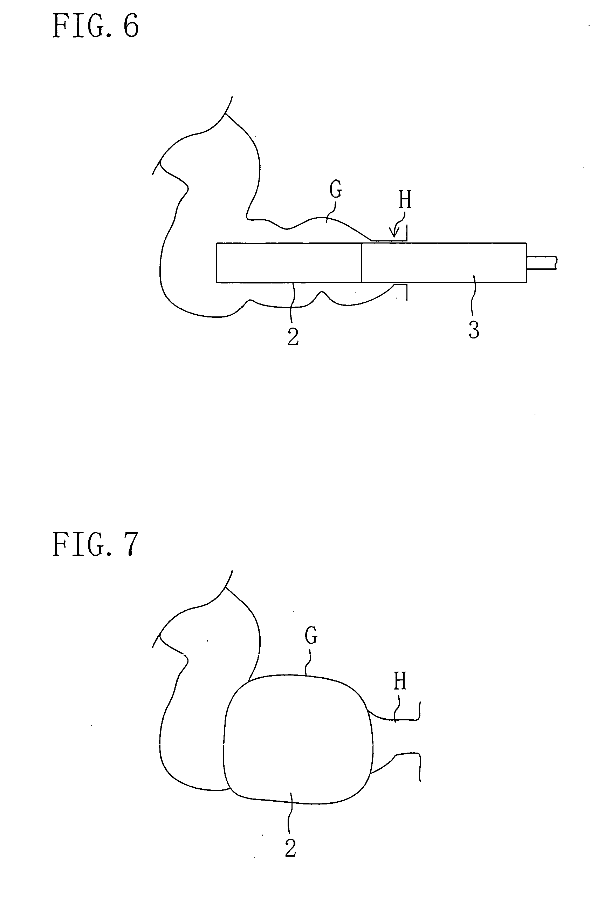

With reference to Figures **6** and **7,** a description will be made of how to insert the expandable sealing member **2** through the anus **H** into the rectum **G** using the body cavity blocking device **1** prepared in the above manner.

A lubricant is applied to the flexible pinnate members **4** and the outer periphery of the cylindrical member **3** and the body cavity blocking device **1** is then inserted, beginning with the flexible pinnate members **4** serving as the insertion head, into the anus **H.** When the cylindrical member **3** has been inserted about halfway, the insertion is halted. Thereafter, the rod member **10** is pushed forward with the rear half of the cylindrical member **3** in hand to extrude the expandable sealing member **2** in the cylindrical member **3** into the rectum **G.** When the expandable sealing member **2** is pushed forward, the flexible pinnate members **4** naturally open outward and the expandable sealing member **2** is gradually forced out of the cylindrical member **3.**

After the expandable sealing member **2** is forced out of the cylindrical member **3,** the cylindrical member **3** is pulled out of the anus **H** with the rear half of the cylindrical member **3** in one hand and the flange **11** of the rod member **10** in the other.

On the other hand, the expandable sealing member **2** pushed in the rectum **G** immediately absorbs body fluids in the rectum **G** so that the superabsorbent fibers rapidly swell in their radial direction. As a result, as shown in Figure 7, the superabsorbent fibers come into tight contact with the inner wall of the rectum **G,** further swell to expand the inner wall and seal the passage of the rectum **G.**

According to the first embodiment, when the worker pushes the rod member **10** to extrude the expandable sealing member **2** into the rectum **G,** he or she can understand in advance how much the rod member **10** should be pushed to extrude the expandable sealing member **2** from the cylindrical member **3.** Therefore, in this embodiment, the rod member **10** is not particularly provided with an indication such a mark or a stopper. However, if necessary, the rod member **10** may be provided with such an indication.

### (Second Embodiment)

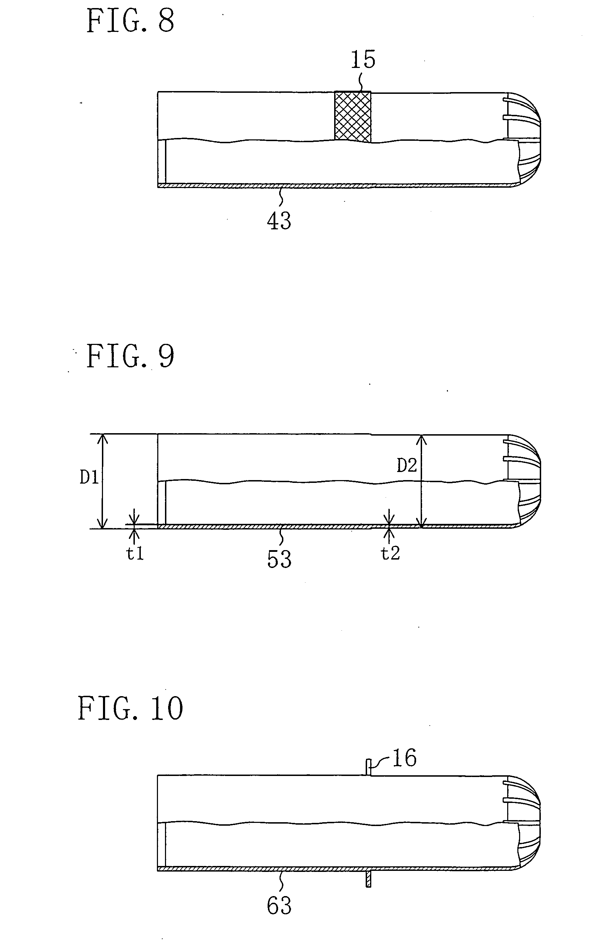

Figure **8** is a view showing the shape of a cylindrical member **43** according to a second embodiment of the present invention. Only different points from the first embodiment will be described here.

In this embodiment, the outer periphery of the cylindrical member **43** is provided with a mark **15** which indicates the position of end of insertion when the cylindrical member **43** is inserted into the anus **H.** In other words, the mark **15** indicates the target distance by which the cylindrical member **43** should be inserted into a body cavity. The mark **15** is formed by giving the cylindrical member **43** a distinct color. Alternatively, a tape may be attached to the cylindrical member **43.** Still alternatively, only that part of the cylindrical member **43** may be given a graining, embossing or striped pattern to distinguish it from the other part or the rear half of the cylindrical member **43** including that part may be given a graining, embossing or striped pattern to distinguish it from the forward half. In such cases, the cylindrical member **43** is hard to slip on (easy to grip) in pulling out it with the rear half thereof in hand, which is advantageous. According to this embodiment, the cylindrical member **43** can be prevented from being more inserted into the body cavity than necessary and also prevented from coming short of insertion. Further, the mark **15** also gives a good indication if the lubricant is applied to the part of the cylindrical member **43** forward of the mark **15.**

### (Third Embodiment)

**Figure 9** is a view showing the shape of a cylindrical member **53** according to a third embodiment of the present invention. Only different points from the first embodiment will be described here.

In this embodiment, the wall thickness **t1** of the rear half of the cylindrical member **53** (the portion thereof closer to the rear end) is set to be larger than the wall thickness **t2** of the forward half thereof (the portion thereof closer to the forward end) and the outer diameter **D1** of the rear half of the cylindrical member **53** is set to be slightly larger than the outer diameter **D2** of the forward half thereof. With this configuration, after the forward half of the cylindrical member **53** is inserted into the anus **H,** the insertion can be stopped when the resistance to insertion becomes slightly large owing to the larger diameter **D1.** Therefore, this embodiment has a merit that the worker can know the degree of insertion by the feeling of insertion of the cylindrical member **53** even if he or she cannot see such a thickness difference. According to this embodiment, the cylindrical member **53** can be prevented from being more inserted into the body cavity than necessary and also prevented from coming short of insertion.

### (Fourth Embodiment)

Figure **10** is a view showing the shape of a cylindrical member **63** according to a fourth embodiment of the present invention. Only different points from the first embodiment will be described here.

In this embodiment, the cylindrical member **63** is provided in the middle of the length with an extended flange member **16** serving as a stopper for restricting the distance of insertion. The extended flange member **16** need only be a mark extending enough to give a feeling of resistance when the cylindrical member **63** is inserted, but need not be extended much. Alternatively, the extended flange member **16** may be provided, not for its portions coming between both the legs when the cylindrical member **63** is inserted in the anus, but only for its portions coming in front and at the back of the legs. According to this embodiment, the cylindrical member **63** can be prevented from being more inserted into the body cavity than necessary and also prevented from coming short of insertion.

The second to fourth embodiments may be employed singly or in combination.

### (Other embodiments)

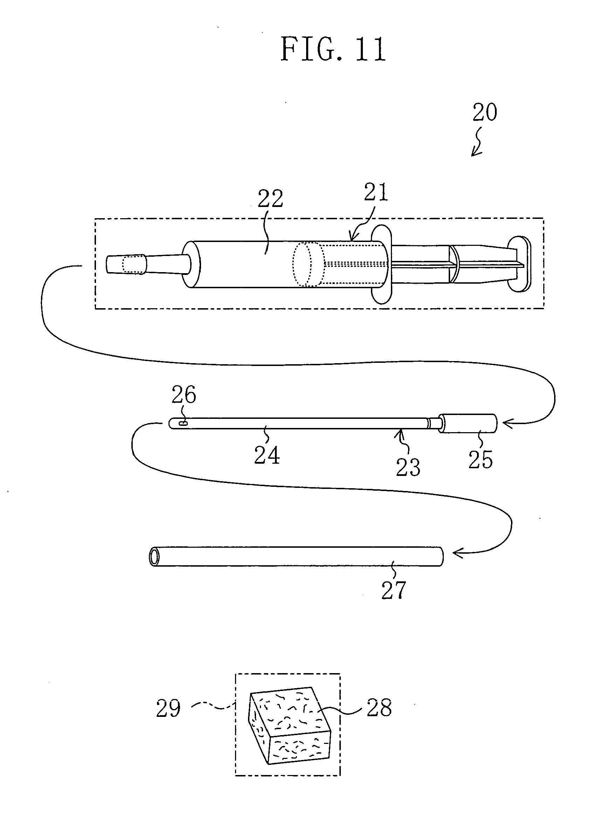

Figure **11** shows a corpse treatment device **20** in which, aside from the above body cavity blocking device **1,** other equipment for sealing body cavities of a corpse, such as the throat and mouth, is collected.

In this example, the corpse treatment device **20** includes a syringe **21** for sealing a throat through a nostril, and an insertion tube **23** which is fittable to the distal end of the syringe **21** and then inserted into the nostril. The syringe **21** is filled with a jelly substance **22** in which superabsorbent resin powder is dispersedly mixed. The insertion tube **23** includes a tube body **24** and a connecting part **25** located at the rear end of the tube body **23** and connectable to the infusion head of the syringe **21,** and has a tube opening **26** located at the distal end of the tube body **23.** Reference numeral **27** denotes a cover member for covering the insertion tube **23.**

The superabsorbent resin powder is preferably dispersed at 5,000 or more particles/ml in the jelly substance **22,** and the jelly substance **22** preferably contains 0.02 to 0.15 parts by weight of a thickener per 100 parts by weight of solvent and has a viscosity of 8,000 to 40,000 CP. More preferably, superabsorbent fine particles are dispersed at 17,000 to 29,000 particles/ml in the jelly substance **22.** Still more preferably, superabsorbent microparticles formed of 20 to 150 mesh particles are dispersed at 18,000 to 25,000 particles/ml in the jelly substance **22.**

Preferably, the jelly substance **22** in the syringe **21** contains at least **21** of ethylene glycol, propylene glycol, diethylene glycol, methanol, ethanol and glycerin, also contains 0.01 to 1.0 parts by weight of an acrylic acid polymer and 0.03 to 0.7 parts by weight of a neutralizer per 100 parts by weight of an alcohol-based principal component, has a viscosity of 8,000 to 40,000 CP and has a PH of 6 to 8. The present invention can employ, as structures of the syringe **21** filled with the jelly substance **22** and the insertion tube **23** and the composition of the jelly substance **22,** those disclosed in Japanese Unexamined Patent Publication No. 2002-275001. Therefore, a further description thereof will not be given here.

Reference numeral **28** denotes a cotton piece, which will be put in a mouth. By putting such a cotton piece **28** in the mouth, body fluids such as saliva can be definitely prevented from leaking out. In addition, when two such cotton pieces **28** are positioned in both cheeks, respectively, the cotton pieces swelled by absorbing body fluids exhibit a good function to fair the shapes of both cheeks. In particular, when dentures are taken out of the corpse of an old person, the cheeks collapse, leading to a poor looking face. However, when the cotton pieces **28** are positioned within the cheeks, they appropriately swell and hence the cheeks can be. kept in appropriate shapes. The cotton piece **28** is not limited to a pure cotton piece. Preferably, 0.5 to 2.0 g of jelly substance is contained in a cotton piece of 4cm by 4cm. In this case, four to five plies of cotton pieces of 4cm by 4cm are preferably prepared. The cotton piece **28** is contained in a container **29** formed of a sealed bag. The body cavity blocking device **1,** the syringe **21,** the insertion tube **23,** the cover member **27** and the cotton piece **28** is contained in an unshown bag. Thus, all necessary items are kept on hand to prevent leakage of body fluids from cavities of a corpse in treating the corpse, particularly, all items for treating cavities in a lower body, such as the anus or virginal cavity, the throat and the mouth are kept on hand. Therefore, the treatment for preventing leakage of body fluids from a corpse can be successfully implemented in a short time.

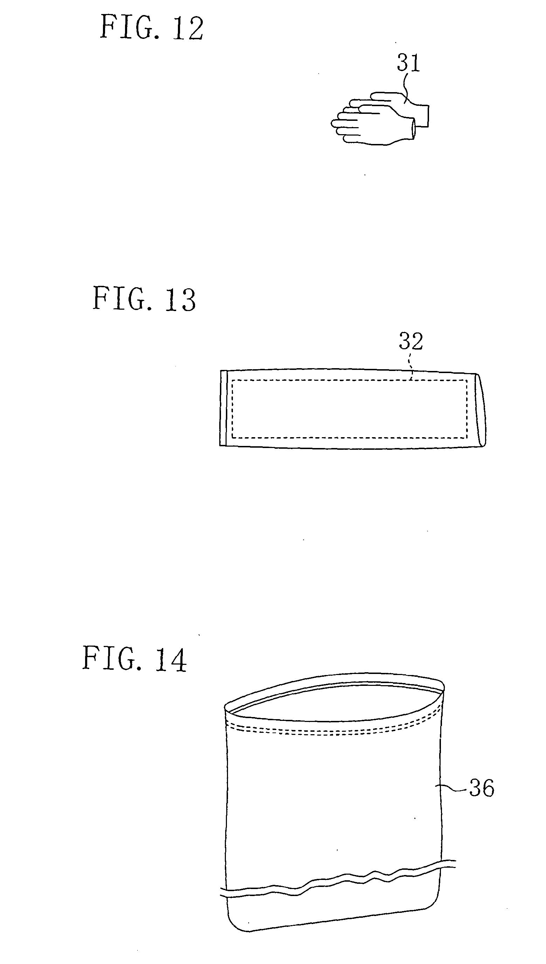

Figures **12** to **15** show equipment that may be additionally contained in the bag for containing the corpse treatment device of the present invention.

Figure **12** shows rubber gloves **31,** which are used in handling equipment of the corpse treatment device, such as a body cavity blocking device **1** and a syringe **21,** and handling the corpse. The addition of the rubber gloves **31** eliminates the need to look for necessary gloves for sanitation of a worker and surely prevents infection or the like to increase safety.

Figure **13** shows a paper-made wet tissue **32** for wiping hands, which includes chlorine dioxide as a disinfectant. This is used by a worker during or after treating a corpse in order to simply wipe his/her hands. The wet tissue **32** for wiping hands is contained in a plastic sealed bag. The addition of the wet tissue **32** for wiping hands prevents infection to increase safety.

Figure **14** shows a container bag **36,** which is for containing the cylindrical member **3,** the syringe **21,** the insertion tube **23,** the container **29,** the cover member **27** and the like after treating a corpse. The container bag **36** is subjected to anti-leaching treatment to avoid leaching of body fluids and the like of the corpse. Since these components are contained in the container bag **36,** they are prevented from being scattered and causing contamination and infection.

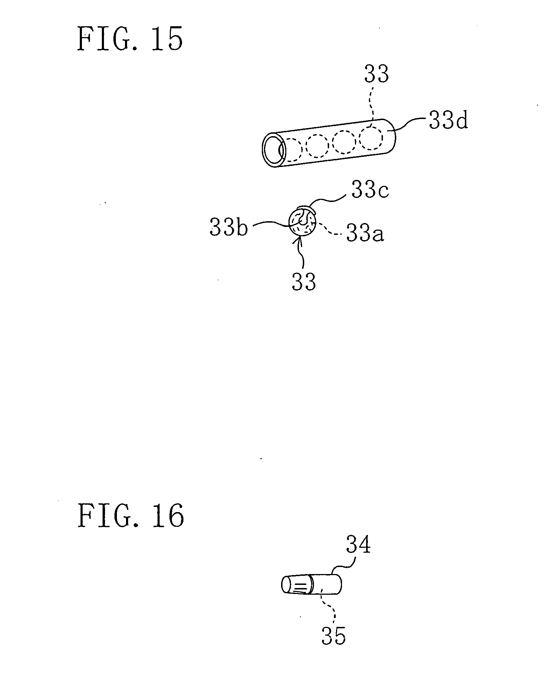

Figure **15** shows sealing members **33** for use as earplugs or nose plugs. Four sealing members **33** are provided in total, two as earplugs and two as nose plugs. Each sealing member **33** is made of a spherical cotton material **33a,** which contains, in its center, a jelly substance **33b** containing superabsorbent resin, namely, a jelly substance identical to the jelly substance **22** in the syringe **21.** Reference numeral **33c** denotes a cap member. The sealing members **33** are contained in a cylindrical, plastic, sealed bag **33d.** The addition of the sealing members **33** further securely prevents body fluids from leaking out. Merely a small amount of body fluid leaks from an ear, and a very small amount of body fluid leaks from a nose when the aforementioned body fluid anti-leakage treatment for a throat is performed. Therefore, a sealing member simply made of a cotton material **33b** may be used as a simple type of sealing member. Though in this embodiment the sealing member **33** is contained in the cylindrical, plastic, sealed bag **33d,** it may be contained in the plastic sealed bag **29** together with the cotton piece **28** for a mouth.

Figure **16** shows a lubricant **35** and a vessel **34** therefor. When the lubricant **35** is applied to the outer surface of the cylindrical member 3 and the insertion tube **23,** they can be smoothly inserted into a body cavity of a corpse. If the lubricant **35** is prepared together with the corpse treatment device and its equipment in this manner, the oblivion of its application can be prevented thereby saving the trouble of looking for lubricant.

Further, necessary items for treating a corpse, such as gauze, absorbent cotton, a disinfectant, a deodorant and a diaper, may be prepared together in a kit. Thus, the work for corpse treatment can be smoothly carried out. Furthermore, another additional equipment may be added, such as a female urethra sealing member, a deodorant, face gauge for a corpse, a band for fixing the jaw of a corpse, a band for joining the hands of a corpse and the like.

A preferable superabsorbent resin-containing jelly substance to be inserted into a throat is one disclosed in Japanese Unexamined Patent Publication No. 2002-275001 and, therefore, its detailed description will not be given here. For example, glycerin or vaseline is used at the lubricant.

The sealing member for an earplug and a nose plug preferably contains, in the cotton material, 0 to 1.5 mg of jelly substance containing superabsorbent resin. The cotton piece for a mouth preferably contains 0.5 to 2.0 g of jelly substance per 4cm by 4cm piece. These jelly substances used are preferably the same as the jelly substance for a throat.

The expandable sealing member of the body cavity blocking member may contain at least one of a deodorant and a disinfectant. Likewise, the jelly substance in the syringe for a throat may contain at least one of a deodorant and a disinfectant. Preferably, a disinfectant is permeated into the paper sheet for wiping hands.

### Industrial Applicability

As described so far, the corpse body cavity blocking device of the present invention can be used by even less experienced persons to easily carry out the work for preventing leakage of body fluids from a corpse and, therefore, is useful for corpse treatment.

## Claims

1. A corpse body cavity blocking device comprising an expandable sealing member and an insertion member for insertion of the expandable sealing member into a body cavity of a corpse, wherein
the expandable sealing member is a molded object obtained by bundling fiber elements of double-layer structure into a substantially columnar shape and is configured, when inserted into the body cavity of the corpse, to absorb body fluids to expand thereby sealing the passage of the body cavity and preventing the leakage of the body fluids,
each of the fiber elements includes an inner layer which is a core made of an acrylic fiber and an outer layer which is formed to surround the inner layer and has a higher water-absorbability and higher swellability than the inner layer, and has the property of absorbing body fluids in the body cavity to expand up to 2.5 or more times the diameter thereof,
the insertion member comprises:
a cylindrical member having an insertion head with which the cylindrical member is first inserted into the body cavity and containing the expandable sealing member slidably placed in the cylindrical member;
a disc-shaped push member placed in the cylindrical member and at one end of the expandable sealing member to slide inside the cylindrical member in the axial direction of the cylindrical member; and
a rod member provided at the opposite side of the disc-shaped push member to the expandable sealing member to cause axial sliding movement of the disc-shaped push member inside the cylindrical member,
the insertion head of the cylindrical member is provided with a plurality of flexible pinnate members formed to extend toward the central axis of the cylindrical member while curving,
the rear end of the cylindrical member is provided integrally with a resistant part which extends from the inner surface of the cylindrical member toward the central axis thereof and provides a resistance to the sliding out of the disc-shaped push member from the rear end of the cylindrical member, and
the rod member is longer than the cylindrical member.

2. The corpse body cavity blocking device of claim 1, wherein
the outer layer of each of the fiber elements is made of hydrophilic cross-linked polymer obtained by hydrophilically cross-linking an acrylic fiber, and
each of the fiber elements has the property of absorbing body fluids to expand up to five or more times the diameter thereof and has a degree of water swelling of 5 to 200 cc/g.

3. The corpse body cavity blocking device of claim 1 or 2, wherein the resistant part is a raised part formed to extend 0.05 to 0.5 mm from the inner surface of the cylindrical member toward the central axis thereof.

4. The corpse body cavity blocking device of any one of claims 1 to 3, wherein a flange for the pushing of the rod member is provided at the end of the rod member opposite to the disc-shaped push member.

5. The corpse body cavity blocking device of claim 4, wherein the flange has the same shape as the disc-shaped push member.

6. The corpse body cavity blocking device of any one of claims 1 to 5, wherein
the disc-shaped push member comprises a disc part contacting the expandable sealing member and a ring part slidably contacting the inner surface of the cylindrical member,
the ring part extends from the peripheral edge of the disc part toward the rear end of the cylindrical member, and
the connecting part between the disc part and the ring part is chamfered.

7. The corpse body cavity blocking device of any one of claims 1 to 6, wherein
the disc-shaped push member has a fitting projection formed integrally on the surface thereof opposite to the surface thereof contacting the expandable sealing member, and
the rod member has a cylinder whose outer diameter is smaller than the inner diameter of the cylindrical member so that the fitting projection is fitted into the inner surface of an end part of the cylinder.

8. The corpse body cavity blocking device of any one of claims 1 to 7, wherein the outer periphery of the cylindrical member is formed with a mark indicating a target distance by which the cylindrical member should be inserted into the body cavity.

9. The corpse body cavity blocking device of any one of claims 1 to 7, wherein
the cylindrical member is formed to have a larger wall thickness in a portion close to the rear end thereof than in a portion close to the insertion head thereof, and
the portion of the cylindrical member close to the insertion head is formed to have the same inner diameter as, but a smaller outer diameter than the portion of the cylindrical member close to the rear end.

10. The corpse body cavity blocking device of any one of claims 1 to 7, wherein the outer periphery of the cylindrical member is provided with a stopper for restricting the distance of insertion of the cylindrical member.

11. The corpse body cavity blocking device of any one of claims 1 to 10, wherein
an opening is formed at the insertion head of the cylindrical member, and
the diameter of the opening is 0.7 to 0.3 times as large as the inner diameter of the cylindrical member.

12. A corpse treatment device comprising:
a body cavity blocking device of any one of claims 1 to 11;
a syringe filled with a jelly substance which contains superabsorbent resin and is to be infused into the throat of a corpse;
an insertion tube connectable to an infusion head of the syringe;
a cover member for covering the insertion tube;
a cotton piece for a mouth to which a jelly substance containing superabsorbent resin is adhered; and
a container for containing the body cavity blocking device, the syringe, the insertion tube, the cover member and the cotton piece,
wherein the insertion tube comprises: a tube body which is made of flexible synthetic resin and configured to be inserted through a nostril into a throat; and a connecting part provided at the rear end of the tube body and connectable to the syringe, and has a tube opening formed at the front end of the tube body.

13. The corpse treatment device of claim 12, wherein gloves for handling a corpse are further contained in the container.

14. The corpse treatment device of claim 12 or 13, wherein a paper sheet for wiping hands is further contained in the container.

15. The corpse treatment device of any one of claims 12 to 14, wherein sealing members for an earplug and a nose plug are further contained in the container.

16. The corpse treatment device of any one of claims 12 to 15, wherein a vessel containing a lubricant is further contained in the container.

17. The corpse treatment device of any one of claims 12 to 16, wherein a container bag for containing the used components including the body cavity blocking device, the syringe, the insertion tube and the cover member is further contained in the container.
